# NEW EUROPEAN PATENT SPECIFICATION

(11) **EP 2 793 595 B2**
(45) Date of publication and mention of the opposition decision: **29.05.2024**
(45) Mention of the grant of the patent: 10.04.2019
(21) Application number: 12808404.3
(22) Date of filing: 21.12.2012
(51) Int. Cl.: A23C 19/032, A23C 19/064, A23C 19/072, A23L 33/00, A23L 27/40, A23L 27/00, C12R 1/225, A23C 19/04, C12N 9/88

(54) **METHOD FOR MAKING LOW-SALT CHEESE USING GAD-NEGATIVE CULTURE AND LOW-SALT CHEESE PRODUCED**
VERFAHREN ZUR HERSTELLUNG VON SALZARMEM KÄSE UNTER VERWENDUNG GAD-NEGATIVER KULTUREN UND DEMENTSPRECHEND HERGESTELLTER SALZARMER KÄSE
PROCÉDÉ POUR FABRIQUER DU FROMAGE A FAIBLE TENEUR EN SEL A L'AIDE D'UNE CULTURE GAD-NÉGATIVE ET FROMAGE A FAIBLE TENEUR EN SEL PRODUIT PAR CETTE MÉTHODE

(30) Priority: 23.12.2011 DK 201101005
(43) Date of publication of application: 29.10.2014
(73) Proprietor: Chr. Hansen A/S, 2970 Hoersholm (DK); Københavns Universitet (University of Copenhagen), 1165 Copenhagen K (DK)
(72) Inventor: MOELLER, Kirsten Kastberg, DK-1307 Copenhagen K (DK); RATTRAY, Fergal, DK-2500 Valby (DK); ARDOE, Ylva, 226 50 Lund (SE); HOEIER, Erik, 2500 Valby (DK)
(74) Representative: Hoffmann Eitle
(86) International application number: PCT/EP2012/076744
(87) International publication number: WO 2013/093049

(56) References cited:
- EP-A1- 1 245 157
- WO-A1-99/09838
- WO-A1-2011/031141
- WO-A1-2011/039414
- WO-A2-00/39281
- WO-A2-02/36752
- NL-C2- 2 003 442
- NOMURA MASARU ET AL: "Inactivation of the glutamate decarboxylase gene in Lactococcus lactis subsp. cremoris", APPLIED AND ENVIRONMENTAL MICROBIOLOGY, vol. 66, no. 5, May 2000 (2000-05), pages 2235-2237, XP002693063, ISSN: 0099-2240
- DANNIELL S D ET AL: "MANUFACTURE OF CHEDDAR CHEESE WITH STREPTOCOCCUS-CREMORIS STRAINS SELECTED FOR INSENSITIVITY TO BACTERIO PHAGES IN THE FACTORY ENVIRONMENT", JOURNAL OF DAIRY SCIENCE, vol. 61, no. SUPPL 1, 1978, page 100, XP009167449, ISSN: 0022-0302
- ADRIANO G CRUZ ET AL: "Cheeses with reduced sodium content: Effects on functionality, public health benefits and sensory properties", TRENDS IN FOOD SCIENCE AND TECHNOLOGY, vol. 22, no. 6, June 2011 (2011-06), pages 276-291, XP028385945, ISSN: 0924-2244, DOI: 10.1016/J.TIFS.2011.02.003 [retrieved on 2011-02-22]

## Description

### FIELD OF INVENTION

The present invention relates to a method for producing cheese with good flavor, especially low-salt cheese.

### BACKGROUND OF INVENTION

Cheese is consumed globally in large and increasing quantities (IDF 2010) and contributes to dietary sodium almost exclusively in the form of NaCl (salt, unless otherwise stated), which is traditionally added in varying amounts dependent on the cheese variety (Guinee and Fox 2004). Hence, in view of an efficient sodium reduction strategy, cheese, apart from constituting a direct and often daily source of sodium, plays an important role in defining the level of salty taste generally accepted by the human perception (taste adaptation) (Walsh 2007). Sodium reduction of significant magnitudes, however, represents a great challenge to the cheese industry since the level of salting is tightly balanced to take advantage of multiple direct and indirect functions of salt in cheese. Particularly in mature cheeses, salt has a critical influence on microbial and enzyme activities needed for a series of biochemical ripening events to proceed in a desired direction.

Cheddar cheese represents a widely consumed international cheese type, a fairly high source of sodium (typically in the range of 1.6 to 2.0% w/w salt (Guinee et al. 2008)) and consequently a priority product for sodium reduction. It is therefore not surprising that numerous previous investigations attempted to restore the quality of low-salt Cheddar cheese. In particular, a series of cheese trials were conducted in the 1980's evaluating with varying degrees of success the partial substitution of NaCl by KCl and other chloride salts (Fitzgerald and Buckley 1985; Lindsay et al. 1982; Reddy and Marth 1993).

However, solutions based on mere salt substitution are not acceptable to the customers, and therefore an objective of the present invention is to provide other solutions to producing low-salt cheeses with a sensory quality matching the corresponding cheeses with a normal salt content (around 1.6 to 1.9%) (Table 1).

**Table 1: NaCl levels of various cheese types**

| **Cheese** | **Normal salt (% (w/w))** | | **Low salt (% (w/w))** | |
|---|---|---|---|---|
| | **lower limit (including)** | **upper limit** | **lower limit** | **upper limit (excluding)** |
| Cheddar | 1.5 | 1.9 | 0.7 | 1.5 |
| Emmental | 0.7 | 1.5 | 0.3 | 0.7 |
| Danbo | 1.4 | 1.9 | 0.7 | 1.4 |
| Parmesan | 2.5 | 4.0 | 1.2 | 2.5 |
| Feta | 3.0 | 4.0 | 1.5 | 3.0 |
| Blue cheese | 3.5 | 4.5 | 1.7 | 3.5 |
| Camembert | 2.5 | 3.3 | 1.2 | 2.5 |
| Gouda | 1.5 | 2.0 | 0.7 | 1.5 |
| Edam | 1.5 | 2.0 | 0.7 | 1.5 |
| Mozzarella | 0.7 | 1.5 | 0.3 | 0.7 |

A. G. Cruz et al, "Cheeses with reduced sodium content: Effects on functionality, public health benefits and sensory properties", Trends in Food Science & Technology 22 (2011) 276-291 discloses preparation of low-salt cheese by replacing part of the NaCl with glutamate. In combination with nucleic acids or yeast extract umami taste was synergistically improved.

### SUMMARY OF INVENTION

The present inventors have surprisingly found out that it is possible to obtain low-salt cheeses of high flavor intensity when a glutamate decarboxylase (GAD)-negative starter comprising a strain selected from the group consisting of: CHCC 4356 deposited as DSM 25485, CHCC 4427 deposited as DSM 25486, CHCC 5188 deposited as DMS 25487, CHCC 1916 deposited as DSM 21405 and IG227 deposited as DSM 14797 plus a GAD-negative*Lactobacillus* (*Lb.*) *helveticus* adjunct culture is used. By combining these strains it was possible to significantly boost the level of glutamate in the low-salt cheese and thereby increase the sensory quality. The GAD-negative starter culture ensures that no glutamate is converted to γ-amino butyric acid (GABA) and CO₂. As glutamate imparts a flavor-enhancing efect and compensates for lack of flavor commonly found in low-slt cheese, then it is important that it accumulates to as high concentracions as possible during ripening. Thus, increased accumulation can be achieved by using a GAD-negartive starter culture. Glutamate levels were boosted even further in the cheese when a *Lb*. *helveticus* adjunct culture was also used. The *Lb. helveticus* adjunct was also GAD-negative, but was further characterized by having a high lytic and peptidolytic activity, and thus releasing high levels of glutamate into the cheese. Thus, it is contemplated that both the starter cultures and the *Lb. helveticus* adjunct culture should be GAD-negative in order to get the optimal (highest) flavor intensity.

A further improvement in the sensory quality of low-salt cheese could be achieved with the use of camel chymosin as coagulant. Normal coagulants such as bovine chymosin, microbially-derived coagulants (from *Mucor miehei*) and plant-derived coagulants are problematic in low-salt cheese. The increased water activity in low-salt cheese results in unbalanced ripening due to the high general (unspecific) proteolyic activity of bovine chymosin, microbially-derived coagulants (from *Mucor miehei*) and plant-derived coagulants. This commonly results in the accumulation of undesirable bitter-tasting peptides derived from the hydrolysis of the caseins during ripening. Surprisingly, the inventors were able to use camel chymosin as coagulant in order to reduce the problem of bitterness in low-salt cheeses. Camel chymosin has a much lower general proteolytic activity than that of bovine chymosin, microbially-derived coagulants or plant-derived coagulants, and thus, it was possible to produce a low-salt cheese with markedly lower rates of bitter-tasting peptide formation during ripening. Levels of bitter-tasting peptides were reduced even further in the cheese when camel chymosin was used in combination with the *Lb. helveticus* adjunct due to aforementioned high lytic and peptidolytic activity.

Thus, the best possible low-salt cheese is produced by combining these two concepts: (i) using a GAD-negative starter culture comprising a strain selected from the group consisting of: CHCC 4356 deposited as DSM 25485, CHCC 4427 deposited as DSM 25486, CHCC 5188 deposited as DSM 25487, CHCC 1916 deposited as DSM 21405 and IG227 deposited as DSM 14797 and a GAD-negative *Lb*. *helveticus* adjunct culture for boosting desirable flavor intensity and (ii) using camel chymosin as coagulant and a *Lb. helveticus* adjunct for the reduction of undesirable, bitter off-taste.

In accordance with the surprising findings, the present invention in its broadest scope relates to a method for producing low-salt cheese comprising the following steps:
a) providing a milk substrate;
b) inoculating the milk substrate with a lactic acid bacteria (LAB) starter culture which is Glu decarboxylation (GAD) negative;
c) adding a coagulant to the milk substrate;
d) allowing the resulting product from steps b) and c) to coagulate;
e) using the coagulum from step d) to produce cheese;
f) optionally storing the cheese;

wherein the total GAD activity results inthat below0.05 mM γ-amino butyric acid (GABA) is produced following incubation in a glutamate containing milk medium at 35°C for 24 hours; and
wherein the low-salt cheese is selected from the group consisting of Cheddar containing an amount of salt in the range 0.1 up to 1.5% - excluding 1.5%;
Parmesan containing an amount of salt in the range 0.1 up to 2.5% - excluding 2.5%;
Mozzarella containing an amount of salt in the range 0.1 up to 0.7% - excluding 0.7%;
Emmental containing an amount of salt in the range 0.1 up to 0.7% - excluding 0.7%;
Danbo containing an amount of salt in the range 0.1 up to 1.4% - excluding 1.4%;
Gouda containing an amount of salt in the range 0.1 up to 1.5% - excluding 1.5%;
Edam containing an amount of salt in the range 0.1 up to 1.5% - excluding 1.5%;
Feta-type containing an amount of salt in the range 0.1 up to 3.0% - excluding 3.0%;
Blue cheese containing an amount of salt in the range 0.1 up to 3.5%- excluding 3.5%; and
Camembert containing an amount of salt in the range 0.1 up to 2.5%- excluding 2.5%; and
wherein the method comprises the further step of inoculating the milk substrate with *Lactobacillus helveticus* (*Lb. helveticus*) strain, said strain is GAD-negative; and
wherein a LAB starter culture or a strain is considered to be GAD-negative if the strain or culture produces less than 0.05 mM GABA in milk, as determined according to the following assay: Reconstituted skim milk at 9.5%, boiled, is inoculated with 10E8 cell forming units of the strain/culture per ml of milk and allowed to stand at 35°C for 24 hours - After the 24 hours the pH has dropped below 5.0, and the content of GABA is measured - If the concentration of GABA is lower than 0.05 mM, the strain/culture is considered to be glutamate decarboxylase negative, and wherein the starter culture comprises a strain selected from the group consisting of: CHCC 4356 deposited as DSM 25485, CHCC 4427 deposited as DSM 25486, CHCC 5188 deposited as DSM 25487, CHCC 1916 deposited as DSM 21405 and IG227 deposited as DSM 14797.

Also, the invention relates to a cheese which is obtainable by the method of the invention.

### DETAILED DISCLOSURE

Aspects of the invention are described in the appended claims.

It should be understood the step e) in the methods of the invention comprises further cheese-making procedures. The exact procedure to be used depends on the cheese to be made. Normally the procedure includes one or more of: cutting the coagulum, draining off the whey from the coagulum (curd), scalding (heating) the curd, adding salt to the curd, molding, and ripening (storing), see e.g. Kosikowski and Mistry (1997).

The starter culture may comprise one or more LAB strains, such as from 2 to 20 strains, from 3 to 20 strains or from 4 to 20 strains. It presently preferred that the starter culture contains more than 1 strain, preferably more than 2 different strains, such as more than 3, more than 4, more than 5 or more than 6 different strains.

The starter culture comprises only GAD-negative strains and comprises a strain selected from the group consisting of: CHCC 4356 deposited as DSM 25485, CHCC 4427 deposited as DSM 25486, CHCC 5188 deposited as DSM 25487, CHCC 1916 deposited as DSM 21405 and IG227 deposited as DSM 14797, no GAD-positive strains are used for inoculation of the milk substrate. Further interesting embodiments of the invention are:
- The method of the invention, wherein the total GAD activity results in that below 0.04 or below 0.03 mM GABA is produced following incubation in a glutamate-containing medium at 35°C for 24 hours.
- The method of the invention, wherein the total GAD activity results in that below 0.05 mM GABA (such as below 0.04 or below 0.03 mM) is produced following incubation in a glutamate-containing medium at 35°C for 48 hours.
- The method of the invention, wherein the total GAD activity results in that below 0.05 mM GABA (such as below 0.04 or below 0.03 mM) is produced following incubation in a glutamate containing medium at 35°C for 72 hours.
- The method of the invention, wherein the glutamate-containing medium is milk (e.g. reconstituted skimmed milk or boiled reconstituted skimmed milk).
- The method of the invention, wherein the step c) is performed before, during and/or after step b).
- The method of the invention, wherein the starter culture comprises LAB selected from the group consisting of Lactobacillales which includes *Lactococcus* spp., *Streptococcus* spp., *Lactobacillus* spp., *Leuconostoc* spp., *Pseudoleuconostoc* spp., *Pediococcus* spp., *Brevibacterium* spp., *Bifidobacterium* spp., *Enterococcus* spp. and *Propionibacterium* spp.
- It is presently preferred that the strain is a component of a LAB starter culture comprising 2 to 20 different strains. It is presently also preferred that the strain constitutes 5 to 95%, such as 20 to 95%, of the starter culture, as measured in cellforming units (CFU).
- The method of the invention, wherein the milk substrate is inoculated with 10E5 to 10E12 CFU/mL of the starter culture.

According to the method of the invention the milk substrate is further inoculated with a *Lb. helveticus* strain (such as a strain having a high lytic and peptidolytic activity or a strain having proteolytic activity), said strain is GAD-negative. It should be understood that only one starter culture (which may contain several different strains) is added to the milk substrate together with the *Lb. helveticus* strain. Preferably, no further cultures are added. The adjunct culture is also GAD negative, and the adjunct culture does only comprise GAD-negative strains.
- The method of the invention, wherein the milk substrate is inoculated with 10E5 to 10E12 CFU/mL of the strain.
- The method of the invention, wherein the resulting product of step b) has a GABA content below 0.05 mM, such as below 0.04 mM, below 0.03 mM or below 0.02 mM.
- The method of the invention, wherein the resulting product of step c) has a GABA content below 0.05 mM, such as below 0.04 mM, below 0.03 mM or below 0.02 mM.
- The method of the invention, wherein the resulting product of step d) has a GABA content below 0.05 mM, such as below 0.04 mM, below 0.03 mM or below 0.02 mM.
- The method of the invention, wherein the resulting product of step e) has a GABA content below 0.05 mM, such as below 0.04 mM, below 0.03 mM or below 0.02 mM.
- The method of the invention, wherein the resulting product of step e) has a GABA content below 0.20 mmol/kg, such as below 0.10 mmol/kg, below 0.05 mmol/kg, below 0.04 mmol/kg, below 0.03 mmol/kg or below 0.02 mmol/kg.
- The method of the invention, wherein the resulting product of step f) (after storage for 5 months) has a GABA content below 0.20 mmol/kg, such as below 0.10 mmol/kg, below 0.05 mmol/kg, below 0.04 mmol/kg, below 0.03 mmol/kg or below 0.02 mmol/kg.
- the method of the invention, wherein the resulting product of step f) (after storage for 9 months) has a GABA content below 0.20 mmol/kg, such as below 0.10 mmol/kg, below 0.05 mmol/kg, below 0.04 mmol/kg, below 0.03 mmol/kg or below 0.02 mmol/kg.
- The method of the invention, wherein the cheese is selected from the group consisting of Cheddar, Parmesan, Mozzarella, Emmental, Danbo, Gouda, Edam, Feta-type, blue cheeses, and Camembert.
- The method of the invention, wherein the cheese is a Cheddar cheese.
- The method of the invention, wherein the cheese has a moisture content in the range from 25% to 70%, such as from 25 to 60%.
- The method of the invention, wherein the coagulant is an aspartic protease, such as chymosin or rennet.
- The method of the invention, wherein the coagulant is selected from the group consisting of bovine chymosin (such as CHY-MAX^{®}).
- The method of the invention, wherein the coagulant is selected from the group consisting of camel chymosin (such as CHY-MAX^{®} M).
- The method of the invention, wherein the storing is performed for a period of 1 to 24 months at a temperature in the range of 5 to 20°C.
- The method of the invention, which comprises a packaging step (before, during or after step f)).

In a further aspect, the present invention relates to a cheese, which is obtainable by the method of the invention, such as a Cheddar cheese. Preferred embodiments of this aspect are:
- A cheese which has a GABA content below 0.20 mmol/kg, such as below 0.10 mmol/kg, below 0.05 mmol/kg, below 0.04 mmol/kg, below 0.03 mmol/kg or below 0.02 mmol/kg;
- A cheese which after storage (ripening/maturation) for 5 months has a GABA content below 0.20 mmol/kg, such as below 0.10 mmol/kg, below 0.05 mmol/kg, below 0.04 mmol/kg, below 0.03 mmol/kg or below 0.02 mmol/kg; or
- A cheese which after storage (ripening/maturation) for 9 months has a GABA content below 0.20 mmol/kg, such as below 0.10 mmol/kg, below 0.05 mmol/kg, below 0.04 mmol/kg, below 0.03 mmol/kg or below 0.02 mmol/kg. It is preferred that the cheeses are low-salt cheese, such as low-salt Cheddar cheeses. Normally cheddar cheeses are ripened at a temperation in the range of 8 to 14 degrees C. Presently most preferred is a low-salt Cheddar that has a GABA content below 0.20 mmol/kg if ripened for 5 months at a temperature of 8C.

Disclosed but not part of the invention is a LAB strain, which is GAD-negative selected from the group consisting of CHCC 4356 deposited as DSM 25485, CHCC 4427 deposited as DSM 25486, CHCC 5188 deposited as DSM25487, CHCC 1916 deposited as DSM21405 and IG227 deposited as DSM 14797; or a mutant or a variant thereof, especially a mutant of any of these strains, which is GAD-negative.

Disclosed but not part of the invention is a LAB culture, which is GAD-negative which contains 2 to 20 different LAB strains, such as 2 to 20 or 3 to 20 different LAB strains, such as a culture, which contains
1) a bacterial strain selected from the group consisting of CHCC 4356 deposited as DSM 25485, CHCC 4427 deposited as DSM 25486, CHCC 5188 deposited as DSM 25487, CHCC 1916 deposited as DSM 21405 and IG227 deposited as DSM 14797; and
2) a LAB strain, which is GAD-negative and which is different from the strain in 1).

In this aspect, the LAB culture can contain 1, 2, 3, 4, 5 or more additional strains, which are different from the strains in 1) and 2).

### DEFINITIONS

In the present context, the term "glutamate (Glu) decarboxylation negative" ("GAD-negative") and the term "glutamate decarboxylase negative"refers to strains of bacteria, especially LAB, which are unable to convert the free amino acid, glutamate into γ-amino butyric acid (GABA) and CO₂. Preferred GAD-negative strains produce GABA to a level of less than 0.05 mM when incubated within the range of 15 to 55°C in a glutamate-containing medium. The incubation period may be up to 72 hours, but shorter or longer periods of incubation are also possible. A typical glutamate-containing medium may be milk with or without glutamate supplementation. In cases, where no glutamate supplementation is used, then the natural level of glutamate in the medium serves as the source of glutamate. In addition, glutamate may be released from various protein and peptide components of the medium during incubation. Other media such as MRS, M17 and Brain Heat Infusion medium (BHI) may also be used for the incubation of the bacteria.

More specifically, a strain (or a culture consisting of one or more strains) is considered to be GAD-negative if the strain or culture produces less than 0.05 mM GABA in milk, as determined according to the following assay:
Reconstituted skim milk (9.5%, boiled) is inoculated with 10E8 cell forming units of the strain/culture per mL of milk and allowed to stand at 35°C for 24 hours. After the 24 hours the pH has dropped below 5.0, and the content of GABA is measured. If the concentration of GABA is lower than 0.05 mM, the strain/culture is considered to be glutamate decarboxylase negative. Preferred strains produce less than 0.04 or less than 0.03 mM GABA in the above assay. Other preferred strains produce less than 0.05, less than 0.04 or less than 0.03 mM GABA in an assay as above, after the pH has dropped below 5.0, but when the milk has been incubated for 48 or 72 hours (instead of 24 hours).

Strains (or cultures) of bacteria that are able to produce GABA to a level of greater than or equal to 0.05 mM are in the present context described as "glutamate decarboxylation positive" ("GAD-positive") or "glutamate decarboxylase positive".

As used herein, the term "lactic acid bacteria" (LAB) designates gram-positive, microaerophilic or anaerobic bacteria, which ferment sugars with the production of acids including lactic acid as the predominantly produced acid, acetic acid and propionic acid. The industrially most useful lactic acid bacteria are found within the order "Lactobacillales" which includes *Lactococcus* spp., *Streptococcus* spp., *Lactobacillus* spp., *Leuconostoc* spp., *Pseudoleuconostoc* spp., *Pediococcus* spp., *Brevibacterium* spp., *Enterococcus* spp. and *Propionibacterium* spp. Additionally, lactic acid producing bacteria belonging to the group of the strict anaerobic bacteria, bifidobacteria, i.e. *Bifidobacterium* spp., are generally included in the group of LAB. These are frequently used as food cultures alone or in combination with other lactic acid bacteria.

By the term "cheese" is understood any cheese, including hard, semi-hard and soft cheeses, such as cheeses of the following types: Cheddar, Parmesan, Mozzarella, Emmentaler, Danbo, Gouda, Edam, Feta-type, blue cheeses, Camembert and Brie. The person skilled in the art knows how to convert the coagulum into cheese, methods can be found in the literature, see e.g. Kosikowski and Mistry (1997).The definition of the term "low-salt" in connection with cheese depends on the cheese type and should normally be understood as cheese, which contains NaCl within a concentration range of 0.2 to 1.7% (w/w) (including the range of 0.2 to 1.6%). More specifically, the term low-salt in the present context can also be read from Table 1 and Table 2. From Table 2 it should be understood that a low-salt cheese of Cheddar type preferably contains an amount of salt in the range 0.1 up to 1.5% (w/w), more preferred in the range 0.2 to 1.3% (w/w) and most preferred in the range 0.3 to 1.1% (w/w).

**Table 2: Definition of low-salt related to specific cheese types (% (w/w))**

| **Cheese** | **Preferred Salt range** | | **More Preferred salt range** | | **Most preferred salt range** | |
|---|---|---|---|---|---|---|
| | **lower limit (including)** | **upper limit (excluding)** | **lower limit** | **upper limit** | **lower limit** | **upper limit** |
| Cheddar | 0.1 | 1.5 | 0.2 | 1.3 | 0.3 | 1.1 |
| Parmesan | 0.1 | 2.5 | 0.2 | 2.0 | 0.3 | 2.0 |
| Mozzarella | 0.1 | 0.7 | 0.2 | 0.6 | 0.2 | 0.5 |
| Emmental | 0.1 | 0.7 | 0.2 | 0.6 | 0.2 | 0.5 |
| Danbo | 0.1 | 1.4 | 0.2 | 1.2 | 0.3 | 1.0 |
| Gouda | 0.1 | 1.5 | 0.2 | 1.3 | 0.3 | 1.2 |
| Edam | 0.1 | 1.5 | 0.2 | 1.3 | 0.3 | 1.2 |
| Feta-type | 0.1 | 3.0 | 0.2 | 2.5 | 0.3 | 2.0 |
| Blue cheese | 0.1 | 3.5 | 0.2 | 3.3 | 0.3 | 3.0 |
| Camembert | 0.1 | 2.5 | 0.2 | 2.2 | 0.3 | 2.0 |

In the present context, the term "milk substrate" may be any raw and/or processed milk material that can be subjected to fermentation according to the method of the invention. Thus, useful milk substrates include, but are not limited to, solutions/suspensions of any milk or milk-like products comprising protein, such as whole or low-fat milk, skimmed milk, buttermilk, reconstituted skimmed milk, condensed milk, dried milk, whey, whey permeate, lactose, mother liquid from crystallization of lactose, whey protein concentrate, or cream. Obviously, the milk substrate may originate from any mammal, e.g. being substantially pure mammalian milk, or reconstituted milk. Preferably, at least part of the protein in the milk substrate is proteins naturally occurring in milk, such as casein or whey protein. However, part of the protein may be proteins which are not naturally occurring in milk. Prior to fermentation, the milk substrate may be homogenized and pasteurized according to methods known in the art.

The term "milk" is to be understood as the lacteal secretion obtained by milking any mammal, such as cows, sheep, goats, buffaloes or camels. In a preferred embodiment, the milk is cow's milk. The term milk also comprises soy milk. Optionally, the milk is acidified, e.g. by addition of an acid (such as citric, acetic or lactic acid), or mixed, e.g. with water or cream. The milk may be raw or processed, e.g. by filtering, sterilizing, pasteurizing, homogenizing etc., or it may be reconstituted dried milk. An important example of "bovine milk" according to the present invention is pasteurized cow's milk. It is understood that the milk may be acidified, mixed or processed before, during and/or after the inoculation with bacteria. The term "providing a milk substrate" includes pretreatment of the milk (substrate), such as the processing steps described above.

By the term "camel chymosin" should be understood a chymosin obtainable by the method of EP1334182B1, incl. the chymosin derived from *Camelus dromedarius.* CHY-MAX^{®} M is presently preferred.

"Fermentation" in the methods of the present invention means the conversion of carbohydrates into alcohols or acids through the action of a microorganism. Preferably, fermentation in the methods of the invention comprises conversion of lactose to lactic acid.

LAB, including bacteria of the species Lactobacillus sp. and Streptococcus thermophilus, are normally supplied to the dairy industry either as frozen or freeze-dried cultures for bulk starter propagation or as so-called "Direct Vat Set" (DVS) cultures, intended for direct inoculation into a fermentation vessel or vat for the production of a dairy product, such as a fermented milk product. Such cultures are in general referred to as "starter cultures" or "starters".

In the present context, the term "packaging" (a suitable amount of) the fermented milk in a suitable package relates to the final packaging of the fermented milk to obtain a product that can be ingested by e.g. a person or a group of persons. A suitable package may thus be a bottle or similar, and a suitable amount may be e.g. 10 mL to 5000 mL, but it is presently preferred that the amount in a package is from 50 mL to 1000 mL.

In the present context, the term "mutant" should be understood as a strain derived from a strain of the invention by means of e.g. genetic engineering, radiation and/or chemical treatment. It is preferred that the mutant is a functionally equivalent mutant, e.g. a mutant that has substantially the same, or improved, properties (e.g. regarding glutamate decarboxylase activity (an interesting mutant has decreased activity compared to mother strain), flavor, acidification speed, and/or phage robustness) as the mother strain. Such a mutant is a part of the present invention. Especially, the term "mutant" refers to a strain obtained by subjecting a strain of the invention to any conventionally used mutagenization treatment including treatment with a chemical mutagen such as ethane methane sulphonate (EMS) or N-methyl-N'-nitro-N-nitroguanidine (NTG), UV light or to a spontaneously occurring mutant. A mutant may have been subjected to several mutagenization treatments (a single treatment should be understood one mutagenization step followed by a screening/selection step), but it is presently preferred that no more than 20, or no more than 10, or no more than 5, treatments are carried out. In a presently preferred mutant, less that 5%, or less than 1% or even less than 0.1% of the nucleotides in the bacterial genome have been shifted with another nucleotide, or deleted, compared to the mother strain.

In the present context, the term "variant" should be understood as a strain, which is functionally equivalent to a strain of the invention, e.g. having substantially the same, or improved, properties (e.g. regarding glutamate decarboxylase activity (an interesting variant has decreased activity compared to mother strain), flavor potential, acidification speed and/or phage robustness). Such variants, which may be identified using appropriate screening techniques, are a part of the present invention.

The use of the terms "a", "an", "the" and similar referents in the context of describing the invention (especially in the context of the following claims) is to be construed to cover both the singular and the plural, unless otherwise indicated herein or clearly contradicted by context. The terms "comprising", "having", "including" and "containing" are to be construed as open-ended terms (i.e. meaning "including, but not limited to,"), unless otherwise noted. Recitation of ranges of values herein are merely intended to serve as a shorthand method of referring individually to each separate value falling within the range, unless otherwise indicated herein, and each separate value is incorporated into the specification as if it was individually recited herein. All methods described herein can be performed in any suitable order, unless otherwise indicated herein or otherwise clearly contradicted by context. The use of any and all examples, or exemplary language (e.g. "such as") provided herein, is intended merely to better illuminate the invention and does not pose a limitation on the scope of the invention, unless otherwise claimed. No language in the specification should be construed as indicating any non-claimed element as essential to the practice of the invention.

### EXAMPLES

### Example 1:

### Screening for GAD-negative strains

Strains were screened based on their inability to decarboxylate glutamate by measuring the levels of γ-amino butyric acid (GABA) and glutamate following growth in milk. Two g of frozen culture pellets was dispersed into 200 g of double boiled 9.5% reconstituted skimmed milk. A 0.01% final inoculation rate was prepared by taking 2 g of this dispersion and inoculating into 200 g of double boiled 9.5% reconstituted skimmed milk, previously spiked with 1 mM glutamate (spiking was performed by the addition of 2 mL of a 100 mM glutamate solution to 200 g of 9.5% reconstituted skimmed milk). The cultures were incubated at 30°C for *Lc. lactis* and 37 C for *Lb. helveticus* for 72 hours. After incubation 1.0 g of fermented skim milk was mixed with 1.0 mL of 4% (w/v) TCA and the samples were mixed, and left stand at room temperature for 30 min. The samples were then centrifuged at 15,000 x g for 20 min at 4°C, and 1 mL of supernatant was transferred into a fresh eppendorf, and re-centrifuged at 15,000 x g for 10 min at 4°C. The final supernatant was stored at -50°C until analysis by GC-MS. Free amino acids were determined according to the method of Glastrup and Houlberg (2009).

Using this screening method it was possible to select strains CHCC 1916, IG227, CHCC 4356, CHCC 4427 and CHCC 5188, which clearly exhibited a glutamate decarboxylase negative phenotype. Glutamate decarboxylase negative strains were characterized by their inability to produce GABA in the presence of glutamate (Table 2). Consequently, any of these strains alone or in combination could be used as a starter culture in order to ensure that glutamate conversion to GABA is limited and/or prevented. For the formulation of a phage robust culture, then a combination of GAD-negative strains is preferable. Thus, a phage robust culture with GAD-negative properties was formulated by mixing strains of CHCC 1916, IG227, CHCC 4427 and CHCC 5188.

For cheese application experiments (Example 3), the GAD-negative strains were grown in a milk-based medium, harvested, concentrated and frozen in order to produce a DVS culture. In addition to producing a frozen DVS culture, a freeze-dried culture is also possible. Production of the culture in DVS format is for purposes of convenience during cheese application experiments, but the essential characteristic (i.e. absence of GAD activity) of the culture remains whether it is concentrated or not.

**Table 3: Screening of glutamate decarboxylase-negative strains**

| **Strain or culture** | **Glutamate (mM)** | **γ-amino butyric acid (mM)** | **GAD reaction** |
|---|---|---|---|
| CHCC 1916 | 1.6 | <LOQ | Negative |
| IG227 | 1.6 | <LOQ | Negative |
| CHCC 4356 | 2.0 | <LOQ | Negative |
| CHCC 4360 | 1.4 | <LOQ | Negative |
| CHCC 4427 | 1.6 | <LOQ | Negative |
| CHCC 5188 | 1.4 | <LOQ | Negative |
| R-604 | 0.0 | 0.5 | Positive |
| LH-32 | 4.8 | <LOQ | Negative |
| LOQ (limit of quantification) = 0.01 mM | | | |

For purposes of comparison, the commercial starter culture F-DVS R-604 (Chr. Hansen) was included in the screening experiment. This culture exhibited a GAD-positive phenotype and is consequently unsuitable for cheese trials, where high levels of glutamate are required. Other commercial starter cultures with GAD activity include: F-DVS R-603 (Chr. Hansen), F-DVS R-607 (Chr. Hansen), F-DVS R-608 (Chr. Hansen), CHOOZIT MA 11 (DuPont), CHOOZIT MA 14 (DuPont), CHOOZIT MA 16 (DuPont) and CHOOZIT MA 19 (DuPont).

The screening method was also used in order to select a *Lb. helveticus* adjunct culture (F-DVS LH-32) with no GAD activity. In addition to this property, the screening revealed that the *Lb. helveticus* strain was able to increase the level of glutamate in the milk. Milk was spiked with 1 mM glutamate at the start of the fermentation and after incubation for 72 hours, the level had increase to 4.8 mM. This *Lb. helveticus* culture is commercially available from Chr. Hansen as F-DVS LH-32. Other similar commercial cultures include: F-DVS LHB01 (Chr. Hansen), F-DVS LHB02 (Chr. Hansen), CHOOZIT FLAV 25, (DuPont), CHOOZIT FLAV 54 (DuPont), CHOOZIT HelvA (DuPont), CHOOZIT HelvB (DuPont), CHOOZIT LH 11 (DuPont) and L100 (CSK Food Enrichment).

### Example 2:

### Production of normal-salt and low-salt Cheddar cheeses with reduced bitterness

All cheeses were manufactured in duplicate. A normal level of salt was defined as 1.8% (w/w) and low-salt cheese was defined as 0.8% (w/w).

The trial was set-up according to Table 4, wherein the starter culture is the GAD-positive F-DVS R-604 (Chr. Hansen).

**Table 4: Salt and coagulant variables**

| | **Cheese ID** | **Salt level** | **Coagulant** |
|---|---|---|---|
| 1 | NB1-26-2-4 | Normal (1.8% (w/w)) | Bovine |
| | NB2-26-4-4 | | chymosin |
| 2 | NC1-26-2-5 | Normal (1.8% (w/w)) | Camel chymosin |
| | NC2-26-4-5 | | |
| 3 | LB1-26-2-6 | Low (0.8% (w/w)) | Bovine |
| | LB2-26-4-6 | | chymosin |
| 4 | LC1-26-2-7 | Low (0.8% (w/w)) | Camel chymosin |
| | LC2-26-4-7 | | |
| 1. Normal-salt cheese made with bovine chymosin as coagulant (NB1-26-2-4, NB2-26-4-4) | | | |
| 2. Normal-salt cheese made with camel chymosin as coagulant (NC1-26-2-5, NC2-26-4-5) | | | |
| 3. Low-salt cheese made with bovine chymosin as coagulant (LB1-26-2-6, LB2-26-4-6) | | | |
| 4. Low-salt cheese made with camel chymosin as coagulant (LC1-26-2-7, LC2-26-4-7) | | | |

Cheddar cheese-making trials were undertaken on at the Application and Technology Centre, Chr. Hansen A/S, Hørsholm, Denmark. Open vats of cheese on a 150 L scale were manufactured. Fresh, pasteurised (74°C, 15 s) bovine milk (3.40% fat, 3.25% protein) was delivered on the morning of production from Arla Foods Amba (Christiansfeld Mejericenter, Denmark). The milk was analysed by MilkoScan^{™} (FOSS, Hillerød, Denmark) and standardised to a protein:fat ratio of 0.9 and heated to 31-32°C for cheese-making. Frozen DVS starter (R-604) was added at a rate of 0.012% (w/w). The milk was coagulated by the addition of CHY-EXTRA^{®} (bovine chymosin) at a rate of 0.0061% w/w (600 IMCU·mL⁻¹; Chr. Hansen A/S) or of CHY-MAX^{®} M (camel chymosin) at a rate of 0.0029% (w/w) (1000 IMCU·mL⁻¹; Chr. Hansen A/S). The curd grains were heated to target temperature and cooked at this temperature for variable lengths of time (Table 5).

**Table 5: Settings of variable technological parameters of cheese-making protocols used for manufacture of full fat Cheddar cheese of variable final salt content and equal final moisture content (37.0 to 38.0% (w/w))**

| **Salt level** | **Target salt (% (w/w))** | **Curd grain cutting size (mm³)** | **Cooking (°C/min)** | **Cheddaring^{a} (#_{layers}/min)** | **Chip size (L × W × T) (cm)** | **Salting rate (% (w/w))** |
|---|---|---|---|---|---|---|
| Low | 0.8 | 125 | 40.5/45 | 1/25-3/15-3/15-4/15-6/15-6/15 | 4.0×1.8×2.0 | 0.95 |
| Normal | 1.8 | 1000 | 37.0/5 | 1/20-2/25-3/30-3/15-4/15-6/10 | 1.7×1.1×3.0 | 3.70 |
| Abbreviations: L, length; T, thickness; W, width | | | | | | |
| ^{a} All turning steps are tabled with the number of curd blocks per pile and the time interval of each turn. | | | | | | |

The milled curds were dry-salted in a warm vessel by manually mixing approximately half the amount of salt thoroughly into the curd followed by a few minutes rest before mixing in the remaining salt; except for the lowest rate of salting which was added all at once (Table 5). The salted curds were mixed thoroughly every 5 min over a mellowing period of 20 min to obtain a uniform distribution of the salt; whey drainage during mellowing was confined by a plastic lining of the vessel. Following molding, the curds were pre-pressed at 0.20 MPa for 15 min and then at 0.57 MPa for 17 hours. After pressing, each cheese was cut into 4 equal blocks, which were vacuum packed separately and ripened at 9°C until analysis.

### Cheese compositional analysis

The NaCl content was determined by automated potentiometric endpoint titration of Cl⁻ with AgNO₃ (DL50, Mettler-Toledo A/S, Glostrup, Denmark. Moisture was analyzed gravimetrically by drying to a constant weight at 102°C. Fat and protein were determined by near infrared reflectance spectroscopy using a Cheddar cheese calibration model (FoodScan^{™} Lab, FOSS). The pH was measured potentiometrically (pHC2002, Radiometer Analytical SAS, Lyon, France) in a paste prepared by macerating 10 g of grated cheese in 10 mL of deionised water. Duplicate samples were prepared for the above analyses.

### Peptide analysis

The pH 4.6-soluble peptides were analyzed by reversed phase (RP) HPLC electro spray ionization mass spectrometry. Thawed, grated cheese (10.0 g) was dispersed in 40 mL of 0.5 mol·L⁻¹ Na₃ citrate by stirring in a 45°C water bath for 1 h. After cooling to room temperature, the cheese slurries were diluted to 5% (w/v) with water and stirred for 5 min. Aliquots of the cheese slurries were cooled to 12 °C and adjusted to pH 4.5 by adding 1 mol·L⁻¹ cold HCl with vigorous mixing. Total volumes of 20 mL were centrifuged (2,000 x g, 30 min, 5°C), and the supernatants were filtered (0.20 µm) and stored at -20°C until analysis. Thawed samples were centrifuged (13,000 x g, 3 min, ambient) before injection (30 µl) onto the RP-HPLC (1100 Series, Agilent Technologies ApS) column (Zorbax 300 SB-C18, 2.1 x 150 mm, 5 µm, Agilent Technologies ApS). Separation of the peptides was performed at 40 °C using binary gradient elution by mixing 0.1% (v/v) TFA (for spectroscopy; Merck Chemicals, Darmstadt, Germany) in water (eluent A) with 0.1% (v/v) TFA in acetonitrile:water (90:10 (v/v)) (eluent B) (acetonitrile was Rathburn HPLC Grade S, Mikrolab Aarhus A/S, Højbjerg, Denmark). A linear gradient was generated by increasing the concentration of eluent B from 2 to 55% over 60 min, after which the column was purged with 100% B for 6 min and re-equilibrated at 2% B for 10 min. The flow rate was 0.25 mL·min⁻¹, and detection was at 210 and 280 nm. The instrument was interfaced with Bruker Daltonik MSD Trap Control software, Ver. 5.3 and ChemStation for LC 3D systems, Rev. B.01.03-SR2 (Agilent Technologies ApS) and data were processed in Bruker Daltoniks DataAnalysis software, Ver. 3.3 (Agilent Technologies ApS). The area of selected peaks (A_{210 nm}) was calculated by integration in ChemStation software, Rev. B.01.03 (Agilent Technologies ApS) and used for peak-wise quantitative comparison of the underlying peptide(s) across cheeses.

### Free amino acid analysis

Free amino acid determination of the cheeses was performed by RP-HPLC with o-phthaldialdehyde and fluorenylmethyl chloroformate pre-column derivitisation according to the method of Bütikofer and Ardö (1999).

### Sensory Analysis

Descriptive sensory analysis was performed in collaboration with the research group of Sensory Science, Department of Food Science, University of Copenhagen. The sensory panel consisted of 10 external persons (7 females, 3 males, aged between 22 and 39 years) previously tested, selected and trained according to IS022935-1:IDF99-1 (2009). Four training sessions of 2 hours guided by a panel leader took place on 4 consecutive days and served to further develop a pre-drafted vocabulary until agreement was reached upon a "best shared language" describing the sensory properties experienced within the set of experimental cheeses. Qualitative and quantitative calibration of the panel was achieved through fine-tuning of descriptor and scale definitions, use of reference materials, standardization of evaluation procedures and evaluation of sub-sets of the experimental cheeses. In the following week, 4 evaluation sessions were undertaken on 4 consecutive days. Samples of rind-free cheese (9 x 1.5 x 1.5 cm of which 1.5 cm were cut off) were freshly cut on the day of use, distributed and blinded in transparent plastic beakers with lids and tempered to 14°C during 2 hours prior to presentation to the panel (ISO22935-2:IDF99-2 2009). The normal-salt cheese was selected for serving (blinded) to the panel as an additional "warm-up sample" at the start of each evaluation session, which took place in test rooms complying with ISO8589 (2007). All descriptors were scored on a horizontal 15 cm unstructured line scale anchored at both ends with appropriate extremes and data were collected by FIZZ software, Ver. 2.46B (Biosystemes, Couternon, France). Panelists were provided with unsalted and unflavored crisp bread, peeled cucumber slices and tap water for palate cleansing. All 8 different samples were served and evaluated one by one in randomized order in all 4 sessions (Latin square design, FIZZ, Ver. 2.46B, Biosystemes).

### Characteristics of normal-salt and low-salt cheeses

One of the major defects associated with the production of low-salt cheese is that a serious flavor defect known as "bitterness" develops. Bitterness is due to the accumulation of mainly small to medium-sized, hydrophobic peptides in the cheese above a certain threshold. The accumulation of these peptides is often due to excessive activity of the coagulant in the low-salt environment. The coagulants used to manufacture such cheese have typically been bovine chymosin or acid proteinases from moulds such as *Mucor miehei.* However, recently, it has been shown that camel chymosin can be used to produce cheese. Camel chymosin has a lower general proteolytic activity than bovine chymosin.

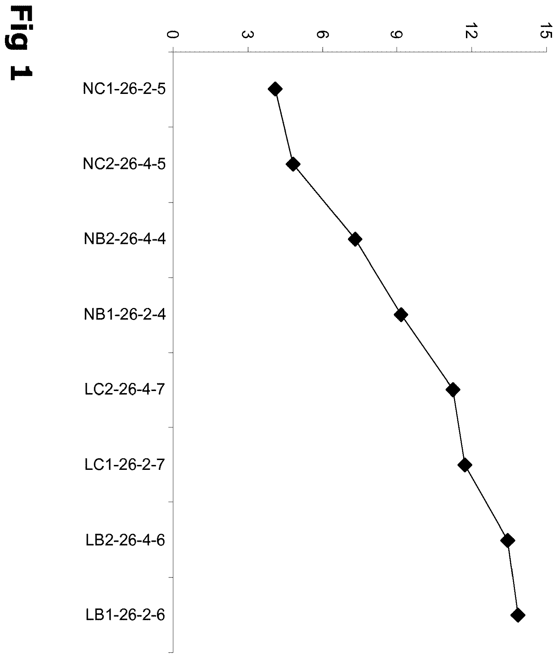

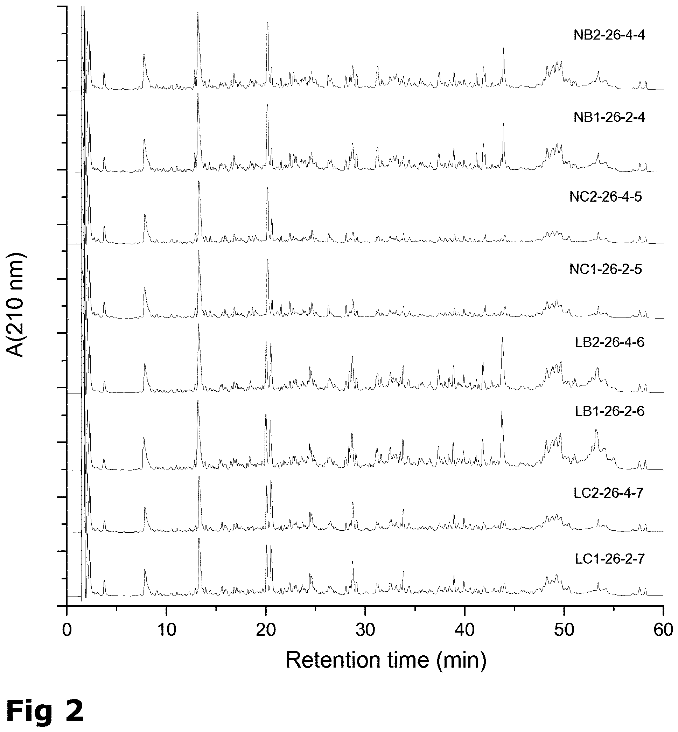

After ripening for 9 months at 9 °C the cheeses were assessed by a trained sensory panel. Cheeses manufactured with low salt and bovine chymosin (LB1-26-2-6, LB2-26-4-6) had the highest bitterness score (Figure 1). This was further illustrated by the highest concentration of hydrophobic peptides in these cheeses (retention time 47-50 min, Figure 2). More specifically, higher concentrations of small to medium-sized, hydrophobic peptides (retention time 35-45 min, Figure 2) were present in the low-salt cheeses with bovine chymosin (LB1-26-2-6, LB2-26-4-6). Surprisingly, it was found that when camel chymosin was used in the manufacture of low-salt cheese (LC1-26-2-7, LC2-26-4-7), the bitterness score was significantly lower than that of the low-salt cheeses manufactured with bovine chymosin (LB1-26-2-6, LB2-26-4-6). This finding is also supported by the chemical analysis of the cheeses which showed lower concentrations of (small to medium-sized) hydrophobic peptides in the low-salt cheeses manufactured with camel chymosin (LC1-26-2-7, LC2-26-4-7).

For normal-salt cheeses, a similar trend regarding the effect of camel chymosin was found. The bitterness score of and level of (small to medium-sized) hydrophobic peptides in the cheeses manufactured with camel chymosin (NC1-26-2-5, NC2-26-4-5) were significantly lower than the corresponding normal-salt cheeses manufactured with bovine chymosin (NB1-26-2-4, NB2-26-4-4). In conclusion, the use of camel chymosin results in lower levels of (small to medium-sized) hydrophobic peptides in, and hence, lower bitterness intensity of both low- and normal-salt cheeses.

### Example 3:

*Production of low-salt Cheddar cheese with reduced bitterness and increased flavor intensity*

All cheeses were manufactured in duplicate. A low-salt cheese was defined as 0.8% (w/w).

The trial was set-up according to Table 6.

**Table 6: Starter culture, adjunct culture and coagulant variables**

| | **Cheese ID** | **Starter culture** | **Adjunct culture** | **Coagulant** |
|---|---|---|---|---|
| 1 | R1-40-4-4 | GAD-positive (F-DVS R-604, Chr. Hansen) | none | Bovine chymosin |
| | R2-40-4-6 | | | |
| 2 | B1-42-1-4 | GAD-negative (F-DVS) | none | Bovine chymosin |
| | B2-42-5-5 | | | |
| 3 | C1-42-1-5 | GAD-negative (F-DVS) | none | Camel chymosin |
| | C2-42-5-4 | | | |
| 4 | BH1-42-1-6 | GAD-negative (F-DVS) | *Lb. helveticus* (F-DVS LH-32, CHr. Hansen) | Bovine chymosin |
| | BH2-42-5-7 | | | |
| 5 | CH1-42-1-7 | GAD-negative (F-DVS) | *Lb. helveticus* (F-DVS LH-32, Chr. Hansen) | Camel chymosin |
| | CH2-42-5-6 | | | |
| 1. GAD-positive starter culture and bovine chymosin as coagulant (R1-40-4, R2-40-4-6) | | | | |
| 2. GAD-negative starter culture and bovine chymosin as coagulant (B1-42-1-4, B2-42-5-5) | | | | |
| 3. GAD-negative starter culture and camel chymosin as coagulant (C1-42-1-5, C2-42-5-4) | | | | |
| 4. GAD-negative starter, *Lb. helveticus* adjunct culture and bovine chymosin as coagulant (BH1-42-1-6, BH2-42-5-7) | | | | |
| 5. GAD-negative starter, *Lb. helveticus* adjunct culture and camel chymosin as coagulant (CH1-42-1-7, CH2-42-5-6) | | | | |

Cheeses were manufactured as described previously (Example 2). F-DVS R-604 was added at a rate of 0.012% (w/w). F-DVS GAD-negative starter culture was added at a rate of 0.02% (w/w). F-DVS LH-32 *Lb. helveticus* adjunct culture was added at a rate of 0.0013% (w/w).

Cheese compositional analysis, pH 4.6-soluble peptide analysis, free amino acid analysis and sensory analysis were performed as described above (Example 2).

### Characteristics of low-salt cheese with reduced bitterness and increased flavor intensity

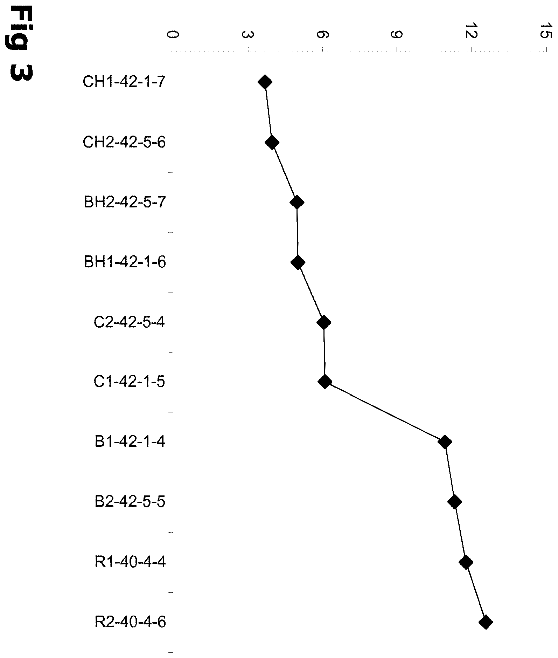

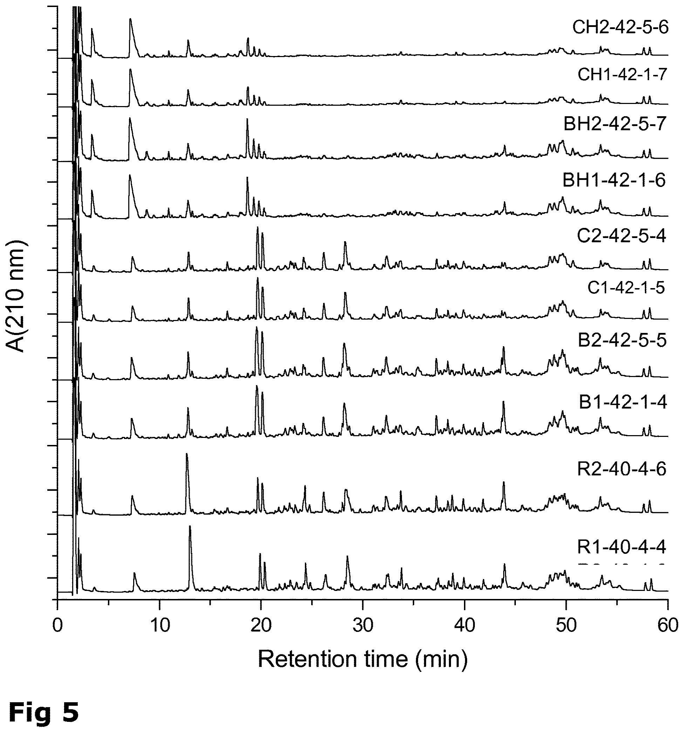

Low-salt cheeses manufactured with the GAD-positive starter (F-DVS R-604) plus bovine chymosin (R1-40-4-4, R2-40-4-6) had the highest bitterness score (Figure 3) and the highest levels of hydrophobic peptides (retention time 47-50 min, Figure 5). More specifically, higher concentrations of small to medium-sized, hydrophobic peptides (retention time 35-45 min, Figure 2) were present in the low-salt cheeses with bovine chymosin (LB1-26-2-6, LB2-26-4-6). Low-salt cheeses produced with the GAD-negative starter plus bovine chymosin (B1-42-1-4, B2-42-5-5) were similar to R1-40-4-4, R2-40-4-6 in terms of having a high bitterness score (Figure 3) and high levels of (small to medium-sized) hydrophobic peptides (retention time 47-50 min, Figure 5). However, low-salt cheeses produced with the GAD-negative starter plus *Lb. helveticus* adjunct culture plus camel chymosin (CH1-42-1-7, CH2-42-5-6) had a significantly lower bitterness score (Figure 3) and lower levels of (small to medium-sized) hydrophobic peptides (retention time 47-50 min, Figure 5). The low-salt cheeses produced with the GAD-negative starter plus *Lb. helveticus* adjunct culture plus bovine chymosin (BH1-42-1-6, BH2-42-5-7) were also found to have a low bitterness score (Figure 3) and low levels of (small to medium-sized) hydrophobic peptides (retention time 47-50 min, Figure 5), but not as low as the low-salt cheeses, where the bovine chymosin was replaced with camel chymosin (CH1-42-1-7, CH2-42-5-6).

Taken in their totality, these results surprisingly show that low-salt cheese with reduced bitterness can be produced by using a GAD-negative starter culture comprising a strain selected from the group consisting of: CHCC 4356 deposited as DSM 25485, CHCC 4427 deposited as DSM 25486, CHCC 5188 deposited as DSM 25487, CHCC 1916 deposited as DSM 21405 and IG227 deposited as DSM 14797 plus a *Lb. helveticus* adjunct culture. A further reduction in bitterness can be achieved if camel chymosin is used instead of bovine chymosin.

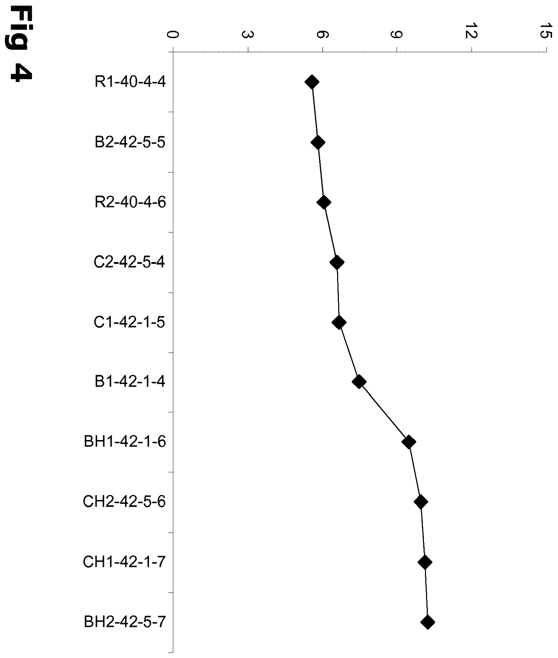

The low-salt cheeses were also assessed for their flavor intensity (umami) by a trained sensory panel. The low-salt cheeses with the highest flavor intensity (Figure 4) were produced using the GAD-negative starter plus *the Lb. helveticus* adjunct culture (BH1-42-1-6, BH2-42-5-7, CH1-42-1-7, CH2-42-5-6). Indeed, cheeses with highest flavor intensity scores (BH1-42-1-6, BH2-42-5-7, CH1-42-1-7, CH2-42-5-6) also had the highest levels of glutamate (28.6 ± 0.9 mmol/kg, after 5 months ripening) (Table 7). In contrast, low-salt cheeses (R1-40-4-4, R2-40-4-6) manufactured with the GAD-positive starter without *Lb. helveticus* adjunct culture were characterised by significantly lower flavor intensities (Figure 4) and glutamate levels (1.2 ± 0.1 mmol/kg, after 5 months ripening) (Table 7). Low-salt cheeses manufactured with the GAD-negative starter and without *Lb. helveticus* adjunct culture (B1-42-1-4, B2-42-5-5, C1-42-1-5, C2-42-5-4) had higher levels of glutamate (4.2 ± 0.2 mmol/kg) than cheeses (R1-40-4-4, R2-40-4-6) made with the GAD-positive (F-DVS R-604) starter (Table 7).

Taken in their totality, these results surprisingly show that low-salt cheese with increased flavor intensity (umami) can be produced by using a GAD-negative starter culture comprising a strain selected from the group consisting of: CHCC 4356 deposited as DSM 25485, CHCC 4427 deposited as DSM 25486, CHCC 5188 deposited as DSM 25487, CHCC 1916 deposited as DSM 21405 and IG227 deposited as DSM 14797 plus a *Lb. helveticus* adjunct culture.

**Table 7: Glutamate levels in cheeses produced with a GAD-negative starter culture, a GAD-negative Lb. helveticus adjunct culture and camel chymosin as coagulant**

| **Cheese** | | **Glutamate (mmol/kg)** | | | | **γ-amino butyric acid (mmol/kg)** | | | |
|---|---|---|---|---|---|---|---|---|---|
| | | **Day 1** | | **Month 5** | | **Day 1** | | **Month 5** | |
| | R1 -40-4-4 | | 1.83 | | 1.31 | | <LOQ | | 3.62 |
| | R2-40-4-6 | | 1.74 | | 1.12 | | <LOQ | | 3.89 |
| | B1-42-1-4 | | 0.70 | | 3.98 | | <LOQ | | <LOQ |
| | B2-42-5-5 | | 0.82 | | 4.41 | | <LOQ | | <LOQ |
| | C1-42-1-5 | | 0.72 | | 4.18 | | <LOQ | | <LOQ |
| | C2-42-5-4 | | 0.85 | | 4.32 | | <LOQ | | <LOQ |
| | BH1-42-1-6 | | 1.79 | | 28.4 | | <LOQ | | <LOQ |
| | BH2-42-5-7 | | 1.65 | | 27.8 | | <LOQ | | <LOQ |
| | CH1-42-1-7 | | 1.73 | | 28.3 | | <LOQ | | <LOQ |
| | CH2-42-5-6 | | 1.74 | | 29.9 | | <LOQ | | <LOQ |
| LOQ (limit of quantificatoion) = 0.2 mmol/kg | | | | | | | | | |

### LEGENDS TO DRAWING

Figure 1 depicts the bitterness score of cheeses produced with a low (L) (0.8% (w/w)) or a normal (N) (1.8% (w/w)) salt content and bovine (B) or camel (C) chymosin. After 9 months ripening.
Figure 2 is RP-HPLC chromatograms of cheeses produced with a low (L) (0.8% (w/w)) or a normal (N) (1.8% (w/w)) salt content and bovine (B) or camel (C) chymosin. After 9 months ripening.
Figure 3 depicts the bitterness score of cheeses produced with a GAD-negative or GAD-positive (R) starter culture, *Lb. helveticus* (H) or no adjunct culture and bovine (B) or camel (C) chymosin as coagulant. After 5 months ripening.
Figure 4 depicts the umami (flavor intensity) score of cheeses produced with a GAD-negative or GAD-positive (R) starter culture, *Lb. helveticus* (H) or no adjunct culture and bovine (B) or camel (C) chymosin as coagulant. After 5 months ripening.
Figure 5 is RP-HPLC chromatograms of cheeses produced with a GAD-negative or GAD-positive (R) starter culture, *Lb. helveticus* (H) or no adjunct culture and bovine (B) or camel (C) chymosin as coagulant. After 5 months ripening.

### DEPOSITS AND EXPERT SOLUTION

The following strains were deposited at Deutsche Sammlung von Mikroorganismen und Zellkulturen GmbH, Inhoffenstr. 7B, D-38124 Braunschweig (DSM):
CHCC 4356 deposited as DSM 25485 on 14^{th} December 2011,
CHCC 4427 deposited as DSM 25486 on 14^{th} December 2011,
CHCC 5188 deposited as DSM 25487 on 14^{th} December 2011,
CHCC 1916 deposited as DSM 21405 on 23^{rd} April 2008 and
IG227 deposited as DSM 14797 on 5^{th} February 2002.

The deposits were made according to the Budapest treaty on the international recognition of the deposit of microorganisms for the purposes of patent procedure.

The Applicant requests that a sample of the deposited microorganism should be made available only to an expert approved by the Applicant.

### REFERENCES

Bütikofer U, Ardö Y (1999) Quantitative determination of free amino acids in cheese. In: IDF General Secretariat (eds) Chemical methods for evaluating proteolysis in cheese maturation (part 2). Bulletin no 337 of the International Dairy Federation. International Dairy Federation, Brussels, Belgium, pp 24-32
Fitzgerald E, Buckley J (1985) Effect of total and partial substitution of sodium chloride on the quality of Cheddar cheese. J Dairy Sci 68:3127-3134
Glastrup J, Houlberg U (2009) Derivatisering med chlorformiater - analyse af aminosyrer med GCMS. Dansk Kemi 90:30-31
Guinee TP, Fox PF (2004) Salt in cheese: physical, chemical and biological aspects. In: Fox PF, McSweeney PLH, Cogan TM, Guinee TP (eds) Cheese: chemistry, physics and microbiology. Volume 1: general aspects. Elsevier Academic Press, San Diego, CA, USA, pp 207-259
Guinee TR, Kilcawley KN, Beresford TP (2008) How variable are retail vintage brands of cheddar cheese in composition and biochemistry? Aust J Dairy Tech 63:50-60
ISO22935-1:IDF99-1 (2009) Milk and milk products - Sensory analysis - Part 1: General guidance for the recruitment, selection, training and monitoring of assessors. International Dairy Federation, Brussels, Belgium
ISO22935-2:2009|IDF99-2 (2009) Milk and milk products - Sensory analysis - Part 2: Recommended methods for sensory evaluation. International Dairy Federation, Brussels, Belgium
IDF (2010) The world dairy situation. Bulletin no 446 of the International Dairy Federation. International Dairy Federation, Brussels, Belgium
ISO8589 (2007) Sensory analysis - General guidance for the design of test rooms. 2 ed. International Organisation for Standardisation, Genève, Switzerland
Kosikowski FV, Mistry VV (1997) Cheese and fermented milk foods. 3rd ed. FV Kosikowski, LCC, Westport, CT, USA
Lindsay RC, Hargett SM, Bush CS (1982) Effect of sodium/potassium (1:1) chloride and low sodium chloride concentrations on quality of Cheddar cheese. J Dairy Sci 65:360-370
Reddy KA, Marth EH (1993) Proteolysis in Cheddar cheese made with sodium chloride, potassium chloride or mixtures of sodium and potassium chloride. Lebensm Wiss Technol 26: 434-442
Walsh C (2007) Consumer responses to low-salt food products. In: Kilcast D, Angus F (eds) Reducing salt in foods: practical strategies. Woodhouse Publishing Limited, Cambridge, pp 124-133

## Claims

1. A method for producing low-salt cheese comprising the following steps:
a) providing a milk substrate;
b) inoculating the milk substrate with a lactic acid bacteria (LAB) starter culture which is Glu decarboxylation (glutamate decarboxylation - GAD) negative;
c) adding a coagulant to the milk substrate;
d) allowing the resulting product from steps b) and c) to coagulate;
e) using the coagulum from step d) to produce cheese;
f) optionally storing the cheese;
wherein the total GAD activity results in that below 0.05 mMγ -amino butyric acid (GABA) is produced following incubation in a glutamate containing milk medium at 35°C for 24 hours;
and wherein the low-salt cheese is selected from the group consisting of Cheddar containing an amount of salt in the range 0.1 up to 1.5% - excluding 1.5%;
Parmesan containing an amount of salt in the range 0.1 up to 2.5% - excluding 2.5%;
Mozzarella containing an amount of salt in the range 0.1 up to 0.7% - excluding 0.7%;
Emmental containing an amount of salt in the range 0.1 up to 0.7% - excluding 0.7%;
Danbo containing an amount of salt in the range 0.1 up to 1.4% - excluding 1.4%;
Gouda containing an amount of salt in the range 0.1 up to 1.5% - excluding 1.5%;
Edam containing an amount of salt in the range 0.1 up to 1.5% - excluding 1.5%;
Feta-type containing an amount of salt in the range 0.1 up to 3.0% - excluding 3.0%;
Blue cheese containing an amount of salt in the range 0.1 up to 3.5% - excluding 3.5%; and
Camembert containing an amount of salt in the range 0.1 up to 2.5% - excluding 2.5%,
and wherein the method comprises the further step of inoculating the milk substrate with *Lactobacillus helveticus* (*Lb. helveticus*) strain, said strain is GAD-negative;
wherein a strain or LAB starter culture is considered to be GAD-negative if the strain or culture produces less than 0.05 mM GABA in milk, as determined according to the following assay:
Reconstituted skim milk, at 9.5%, boiled, is inoculated with 10E8 cell forming units of the strain/culture per mL of milk and allowed to stand at 35°C for 24 hours - After the 24 hours the pH has dropped below 5.0, and the content of GABA is measured - If the concentration of GABA is lower than 0.05 mM, the strain/culture is considered to be glutamate decarboxylase negative
and wherein the starter culture comprises a strain selected from the group consisting of: CHCC 4356 deposited as DSM 25485, CHCC 4427 deposited as DSM 25486, CHCC 5188 deposited as DSM 25487, CHCC 1916 deposited as DSM 21405 and IG227 deposited as DSM 14797.

2. The method of claim 1, wherein the starter culture comprises LAB selected from the group consisting of *Lactococcus* spp., *Leuconostoc* spp., *Pseudoleuconostoc* spp., *Pediococcus* spp., *Brevibacterium* spp., *Bifidobacterium* spp., *Enterococcus* spp. and *Propionibacterium* spp.

3. The method of any preceding claim, wherein the milk substrate is inoculated with 10E5 to 10E12 CFU/mL of the *Lb. helveticus* strain.

4. The method of any preceding claim, wherein the coagulant is selected from the group consisting of camel chymosin such as CHY-MAX^{®} M.

5. The method of the preceding claim, wherein the starter culture comprises one or more LAB strains, such as from 2 to 20 strains, from 3 to 20 strains or from 4 to 20 strains.

6. The method of any preceding claims, wherein the total GAD activity results in that below 0.05 mM GABA is produced following incubation in a glutamate-containing milk medium at 35°C for 48 hours.

7. A method of any preceding claim for producing a cheese, comprising the following steps:
b) inoculating a milk substrate with a LAB starter culture, which is GAD-negative;
c) adding a camel chymosin to the milk substrate;
d) allowing the resulting product from steps b) and c) to coagulate;
e) using the coagulum from step d) to produce low-salt cheese;
f) optionally storing the cheese.

8. A method of any preceding claim for producing a cheese, comprising the following steps:
b) inoculating a milk substrate with a LAB starter culture, which is GAD-negative and a *L b. helveticus* strain such as a strain having a high lytic and peptidolytic activity or a strain having proteolytic activity, which is GADnegative;
c) adding a coagulant to the milk substrate;
d) allowing the resulting product from steps b) and c) to coagulate;
e) using the coagulum from step d) to produce low-salt Cheddar cheese;
f) optionally storing the cheese.

9. The method of any preceding claim, wherein the culture comprises LAB selected from the group consisting of *Lactococcus* spp., *Leuconostoc* spp., *Pseudoleuconostoc* spp., *Pediococcus* spp., *Brevibacterium* spp., *Bifidobacterium* spp., *Enterococcus* spp. and *Propionibacterium* spp., and contains more than 1 strain, preferably more than 2 different strains, such as more than 3, more than 4, more than 5 or more than 6 different strains.

10. The method of the preceding claim, wherein the culture comprises a bacterial strain selected from the group consisting of: CHCC 4356 deposited as DSM 25485, CHCC 4427 deposited as DSM 25486, CHCC 5188 deposited as DSM 25487, CHCC 1916 deposited as DSM 21405 and IG227 deposited as DSM 14797; and mutants or variants thereof.

11. A cheese which is obtainable by the method of any preceding claims, such as a Cheddar cheese.

## Patentansprüche

1. Verfahren zum Herstellen von salzarmem Käse, umfassend die folgenden Schritte:
a) Bereitstellen eines Milchsubstrats;
b) Inokulieren des Milchsubstrats mit einer Milchsäurebakterium (LAB) -Starterkultur, die Glu-Decarboxylierung (Glutamatdecarboxylierung - GAD) -negativ ist;
c) Hinzufügen eines Gerinnungsmittels zu dem Milchsubstrat;
d) Ermöglichen, dass das aus Schritten b) und c) entstandene Produkt gerinnt;
e) Verwenden des Gerinnungsmittels aus Schritt d) zur Herstellung von Käse;
f) wahlweise Lagern des Käses;
wobei die Gesamt-GAD-Aktivität dazu führt, dass weniger als 0,05 mM γ-Aminobuttersäure (GABA) erzeugt wird, gefolgt von Inkubation in einem Glutamat-haltigen Milchmedium bei 35 °C für 24 Stunden;
und
wobei der salzarme Käse ausgewählt ist aus der Gruppe bestehend aus
Cheddar, enthaltend eine Salzmenge im Bereich von 0,1 bis zu 1,5 % - ausschließlich 1,5 %;
Parmesan, enthaltend eine Salzmenge im Bereich von 0,1 bis zu 2,5 % - ausschließlich 2,5 %;
Mozzarella, enthaltend eine Salzmenge im Bereich von 0,1 bis zu 0,7 % - ausschließlich 0,7 %;
Emmentaler, enthaltend eine Salzmenge im Bereich von 0,1 bis zu 0,7 % - ausschließlich 0,7 %;
Danbo, enthaltend eine Salzmenge im Bereich von 0,1 bis zu 1,4 % - ausschließlich 1,4 %;
Gouda, enthaltend eine Salzmenge im Bereich von 0,1 bis zu 1,5 % - ausschließlich 1,5 %;
Edamer, enthaltend eine Salzmenge im Bereich von 0,1 bis zu 1,5 % - ausschließlich 1,5 %;
Fetakäseart, enthaltend eine Salzmenge im Bereich von 0,1 bis zu 3,0 % - ausschließlich 3,0 %;
Blauschimmelkäse, enthaltend eine Salzmenge im Bereich von 0,1 bis zu 3,5 % - ausschließlich 3,5 %; und
Camembert, enthaltend eine Salzmenge im Bereich von 0,1 bis zu 2,5 % - ausschließlich 2,5 %;
und
wobei das Verfahren ferner den Schritt des Inokulierens des Milchsubstrats mit Lactobacillus helveticus (Lb. helveticus) -Stamm umfasst, wobei der Stamm GAD-negativ ist;
wobei ein Stamm oder eine LAB-Starterkultur als GAD-negativ angesehen wird, wenn der Stamm oder die Kultur weniger als 0,05 mM GABA in Milch erzeugt, wie nach dem folgenden Assay bestimmt wird:
rekonstituierte Magermilch zu 9,5 %, gekocht, wird mit 10E8 zellbildenden Einheiten des Stamms/der Kultur pro mL Milch inokuliert und bei 35 °C für 24 Stunden stehen gelassen - Nach den 24 Stunden ist der pH auf unter 5,0 gefallen, und der Gehalt der GABA wird gemessen - Wenn die Konzentration der GABA geringer als 0,05 mM ist, wird der Stamm/die Kultur als Glutamat-Decarboxylase-negativ angesehen,
wobei die Starterkultur einen Stamm umfasst, ausgewählt aus der Gruppe bestehend aus: CHCC 4356 hinterlegt als DSM 25485, CHCC 4427 hinterlegt als DSM 25486, CHCC 5188 hinterlegt als DSM 25487, CHCC 1916 hinterlegt als DSM 21405 und IG227 hinterlegt als DSM 14797.

2. Verfahren nach Anspruch 1, wobei die Starterkultur LAB umfasst, ausgewählt aus der Gruppe bestehend aus Lactococcus spp., Leuconostoc spp., Pseudoleuconostoc spp., Pediococcus spp., Brevibacterium spp., Bifidobacterium spp., Enterococcus spp. und Propionibacterium spp.

3. Verfahren nach einem der vorstehenden Ansprüche, wobei das Milchsubstrat mit 10E5 bis 10E12 CFU/mL des Lb. helveticus Stammes inokuliert wird.

4. Verfahren nach einem der vorstehenden Ansprüche, wobei das Gerinnungsmittel ausgewählt wird aus der Gruppe bestehend aus Kamel-Chymosin, wie zum Beispiel CHY-MAX^{®} M.

5. Verfahren nach einem der vorstehenden Ansprüche, wobei die Starterkultur einen oder mehrere LAB-Stämme umfasst, wie zum Beispiel von 2 bis 20 Stämme, von 3 bis 20 Stämme oder von 4 bis 20 Stämme.

6. Verfahren nach einem der vorstehenden Ansprüche, wobei die Gesamt-GAD Aktivität dazu führt, dass weniger als 0,05 mM GABA erzeugt wird, gefolgt von Inkubation in einem Glutamat-haltigen Milchmedium bei 35 °C für 48 Stunden.

7. Verfahren nach einem der vorstehenden Ansprüche zum Herstellen eines Käses, umfassend die folgenden Schritte:
b) Inokulieren eines Milchsubstrats mit einer LAB-Starterkultur, die GAD-negativ ist;
c) Hinzufügen eines Kamel-Chymosins zu dem Milchsubstrat;
d) Ermöglichen, dass das aus Schritten b) und c) entstandene Produkt gerinnt;
e) Verwenden des Gerinnungsmittels aus Schritt d) zur Herstellung von salzarmem Käse;
f) wahlweise Lagern des Käses.

8. Verfahren nach einem der vorstehenden Ansprüche zum Herstellen eines Käses, umfassend die folgenden Schritte:
b) Inokulieren eines Milchsubstrats mit einer LAB-Starterkultur, die GAD-negativ ist, und einem LB. helveticus Stamm, wie zum Beispiel einem Stamm mit einer hohen lytischen und peptidolytischen Aktivität oder einem Stamm mit proteolytischer Aktivität, der GAD-negativ ist;
c) Hinzufügen eines Gerinnungsmittels zu dem Milchsubstrat;
d) Ermöglichen, dass das aus Schritten b) und c) entstandene Produkt gerinnt;
e) Verwenden des Gerinnungsmittels aus Schritt d) zur Herstellung von salzarmem Cheddarkäse;
f) wahlweise Lagern des Käses.

9. Verfahren nach einem der vorstehenden Ansprüche, wobei die Kultur LAB umfasst, ausgewählt aus der Gruppe bestehend aus Lactococcus spp., Leuconostoc spp., Pseudoleuconostoc spp., Pediococcus spp., Brevibacterium spp., Bifidobacterium spp., Enterococcus spp. und Propionibacterium spp., und mehr als 1 Stamm enthält, vorzugsweise mehr als 2 unterschiedliche Stämme, wie zum Beispiel mehr als 3, mehr als 4, mehr als 5 oder mehr als 6 unterschiedliche Stämme.

10. Verfahren nach einem der vorstehenden Ansprüche, wobei die Kultur einen Bakterienstamm umfasst, ausgewählt aus der Gruppe bestehend aus: CHCC 4356 hinterlegt als DSM 25485, CHCC 4427 hinterlegt als DSM 25486, CHCC 5188 hinterlegt als DSM 25487, CHCC 1916 hinterlegt als DSM 21405 und IG227 hinterlegt als DSM 14797; und Mutanten und Varianten davon.

11. Käse, der durch das Verfahren nach einem der vorstehenden Ansprüche erhältlich ist, wie zum Beispiel ein Cheddarkäse.

## Revendications

1. Procédé de production d'un fromage à faible teneur en sel comprenant les étapes suivantes :
a) une fourniture d'un substrat de lait ;
b) une inoculation du substrat de lait avec une culture de départ de bactéries d'acide lactique (LAB) qui est négative à la décarboxylation de Glu (décarboxylation du glutamate - GAD) ;
c) un ajout d'un coagulant au substrat de lait ;
d) le fait de permettre au produit résultant issu des étapes b) et c) de coaguler ;
e) une utilisation du coagulum issu de l'étape d) pour produire du fromage ;
f) facultativement un stockage du fromage ;
dans lequel l'activité de GAD totale se traduit par le fait qu'en dessous de 0,05 mM l'acide γ-aminobutyrique (GABA) est produit à la suite d'une incubation dans un milieu de lait contenant du glutamate à 35 °C pendant 24 heures ;
et dans lequel le fromage à faible teneur en sel est sélectionné parmi le groupe consistant en
du cheddar contenant une quantité de sel dans la plage de 0,1 à 1,5 % - excluant 1,5%;
du parmesan contenant une quantité de sel dans la plage de 0,1 à 2,5 % - excluant 2,5 % ;
de la mozzarella contenant une quantité de sel dans la plage de 0,1 à 0,7 % - excluant 0,7 % ;
de l'emmental contenant une quantité de sel dans la plage de 0,1 à 0,7 % - excluant 0,7 % ;
du danbo contenant une quantité de sel dans la plage de 0,1 à 1,4 % - excluant 1,4 % ;
du gouda contenant une quantité de sel dans la plage de 0,1 à 1,5 % - excluant 1,5 % ;
de l'édam contenant une quantité de sel dans la plage de 0,1 à 1,5 % - excluant 1,5 % ;
du fromage de type fêta contenant une quantité de sel dans la plage de 0,1 à 3,0 % - excluant 3,0 % ;
du fromage bleu contenant une quantité de sel dans la plage de 0,1 à 3,5 % - excluant 3,5 % ; et
du camembert contenant une quantité de sel dans la plage de 0,1 à 2,5 % - excluant 2,5 %,
et dans lequel le procédé comprend l'étape supplémentaire consistant à inoculer le substrat de lait avec une souche de *Lactobacillus helveticus (Lb. helveticus)*, ladite souche étant négative à la GAD ;
dans lequel une souche ou culture de départ de LAB est considérée comme négative à la GAD si la souche ou culture produit moins de 0,05 mM de GABA dans le lait, comme déterminé selon l'essai suivant :
Du lait écrémé reconstitué, bouilli à 9,5 %, est inoculé avec 10E8 unités formant des cellules de la souche/culture par ml de lait et mis au repos à 35 °C pendant 24 heures - Au bout des 24 heures le pH est tombé en dessous de 5,0 et la teneur en GABA est mesurée - Si la concentration en GABA est inférieure à 0,05 mM, la souche/culture est considérée comme négative à la décarboxylase du glutamate.
et dans lequel la culture de départ comprend une souche sélectionnée parmi le groupe consistant en : CHCC 4356 déposée en tant que DSM 25485, CHCC 4427 déposée en tant que DSM 25486, CHCC 5188 déposée en tant que DSM 25487, CHCC 1916 déposée en tant que DSM 21405 et IG227 déposée en tant que DSM 14797.

2. Procédé selon la revendication 1, dans lequel la culture de départ comprend des LAB sélectionnées parmi le groupe consistant en *Lactococcus* spp., *Leuconostoc* spp., *Pseudoleuconostoc* spp., *Pediococcus* spp., *Brevibacterium* spp., *Bifidobacterium* spp., *Enterococcus* spp. et *Propionibacterium* spp.

3. Procédé selon une quelconque revendication précédente, dans lequel le substrat de lait est inoculé avec 10E5 à 10E12 UFC/ml de la souche de *Lb. helveticus.*

4. Procédé selon une quelconque revendication précédente, dans lequel le coagulant est sélectionné parmi le groupe consistant en de la chymosine de chameau telle que CHY-MAX^{®} M.

5. Procédé selon la revendication précédente, dans lequel la culture de départ comprend une ou plusieurs souches de LAB, comme de 2 à 20 souches, de 3 à 20 souches ou de 4 à 20 souches.

6. Procédé selon une quelconque des revendications précédentes, dans lequel l'activité de GAD totale se traduit par le fait qu'en dessous de 0,05 mM du GABA est produit à la suite de l'incubation dans un milieu de lait contenant du glutamate à 35 °C pendant 48 heures.

7. Procédé selon une quelconque revendication précédente pour produire un fromage, comprenant les étapes suivantes :
b) une inoculation d'un substrat de lait avec une culture de départ de LAB, qui est négative à la GAD ;
c) un ajout d'une chymosine de chameau au substrat de lait ;
d) le fait de permettre au produit résultant issu des étapes b) et c) de coaguler ;
e) une utilisation du coagulum issu de l'étape d) pour produire du fromage à faible teneur en sel ;
f) facultativement un stockage du fromage.

8. Procédé selon une quelconque revendication précédente pour produire un fromage, comprenant les étapes suivantes :
b) une inoculation d'un substrat de lait avec une culture de départ de LAB, qui est négative à la GAD et une souche de *Lb. helveticus* telle qu'une souche présentant une activité lytique et peptidolytique élevée ou une souche présentant une activité protéolytique, qui est négative à la GAD ;
c) un ajout d'un coagulant au substrat de lait ;
d) le fait de permettre au produit résultant issu des étapes b) et c) de coaguler ;
e) une utilisation du coagulum issu de l'étape d) pour produire du fromage cheddar à faible teneur en sel ;
f) facultativement un stockage du fromage.

9. Procédé selon une quelconque revendication précédente, dans lequel la culture comprend des LAB sélectionnées parmi le groupe consistant en *Lactococcus* spp., *Leuconostoc* spp., *Pseudoleuconostoc* spp., *Pediococcus* spp., *Brevibacterium* spp., *Bifidobacterium* spp., *Enterococcus* spp. et *Propionibacterium* spp., et contient plus de 1 souche, de préférence plus de 2 souches différentes, comme plus de 3, plus de 4, plus de 5 ou plus de 6 souches différentes.

10. Procédé selon la revendication précédente, dans lequel la culture comprend une souche bactérienne sélectionnée parmi le groupe consistant en : CHCC 4356 déposée en tant que DSM 25485, CHCC 4427 déposée en tant que DSM 25486, CHCC 5188 déposée en tant que DSM 25487, CHCC 1916 déposée en tant que DSM 21405 et IG227 déposée en tant que DSM 14797 ; et des mutants ou variantes de celle-ci.

11. Fromage qui peut être obtenu par le procédé selon une quelconque des revendications précédentes, tel qu'un fromage cheddar.
